(19) Europäisches Patentamt
European Patent Office
Office européen des brevets

(11) Numéro de publication: **0 475 801 B1**

(12) # FASCICULE DE BREVET EUROPEEN

(45) Date de publication de fascicule du brevet: **09.11.94**

(51) Int. Cl.5: **C07D 333/10**

(21) Numéro de dépôt: **91402221.5**

(22) Date de dépôt: **09.08.91**

(54) **Procédé de fabrication du thiophène.**

(30) Priorité: **29.08.90 FR 9010762**

(43) Date de publication de la demande:
**18.03.92 Bulletin 92/12**

(45) Mention de la délivrance du brevet:
**09.11.94 Bulletin 94/45**

(84) Etats contractants désignés:
**AT BE CH DE DK ES FR GB GR IT LI LU NL SE**

(56) Documents cités:
**FR-A- 1 256 349**
**US-A- 2 440 671**
**US-A- 2 694 075**

**CHEMICAL ABSTRACTS, vol. 93, no. 13, 29 septembre 1980, page 600, abrégé no.131779d, Columbus, Ohio, US; T.S. SUKHAREVA et al.: "Study of the mechanism of thiopene formation in the presence of sulfide catalysts",**

(73) Titulaire: **SOCIETE NATIONALE ELF AQUITAINE (PRODUCTION)**
**Tour Elf,**
**2, Place de la Coupole,**
**La Défense 6**
**F-92400 Courbevoie (FR)**

(72) Inventeur: **Forquy, Christian**
**603 Brighton Circle**
**Devon, PA 19333 (US)**
Inventeur: **Lacroix, Michel**
**27c Chemin de Montribloud**
**F-69009 Lyon (FR)**
Inventeur: **Breysse, Michèle**
**15 Rue Pierre Bourgeois**
**F-69300 Caluire et Cuire (FR)**

(74) Mandataire: **Leboulenger, Jean et al**
**ATOCHEM**
**Département Propriété Industrielle**
**F-92091 Paris la Défense 10 Cédex 42 (FR)**



Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services
(3.10/3.09/3.3.3)

## Description

La présente invention concerne la fabrication de thiophène par déshydrogénation catalytique du tétrahydrothiophène.

Les applications du thiophène en tant qu'intermédiaire pour la synthèse de produits pharmaceutiques (anti-histaminiques, anti-aggrégants plaquettaires, céphalosporines, anti-inflammatoires, etc...), de stabilisants pour polymères ou de colorants (diazo-thiophène) sont nombreuses et en forte croissance.

Il existe de nombreuses méthodes de fabrication du thiophène, les produits de départ étant très variés :

- butène-1 ou butadiène et $CS_2$ (brevet FR 2 139 177),
- butane ou butène-1, $H_2S$ et soufre (brevet FR 2 242 391),
- butanediol et soufre vaporisé (brevet BE 671 591),
- n.butanol ou 1,4-butanediol et $CS_2$ (brevet FR 2 139 177),
- crotonaldéhyde et $H_2S$ (brevet FR 2 375 227),
- furanne et $H_2S$ (brevet BE 623 801).

Ce dernier procédé est industriel, mais il nécessite un approvisionnement bon marché en furanne, ce qui n'est pas le cas dans la majeure partie du monde. Les autres procédés posent des problèmes de sélectivité par suite de la formation de nombreux produits indésirables. De plus, ce sont des procédés catalytiques dans lesquels un cokage rapide des catalyseurs est observé, ce qui nécessite une régénération fréquente.

Un produit de départ, particulièrement attractif pour la fabrication du thiophène, est le tétrahydrothiophène (ci-après désigné par les initiales THT) dont la synthèse industrielle s'effectue à partir du tétrahydrofuranne, une matière première peu coûteuse. Une installation de fabrication de thiophène peut avantageusement être mise à la suite d'une synthèse industrielle de THT à partir de tétrahydrofuranne, sans nécessiter un investissement trop important. De plus, on peut facilement séparer le THT du thiophène ; il n'est donc pas nécessaire de travailler à conversion totale et cela permet d'adapter à la demande la production de l'unité industrielle.

La déshydrogénation du THT a fait l'objet de quelques travaux. Il est en effet connu d'utiliser comme catalyseur pour cette réaction des oxydes d'aluminium et de cobalt ou d'aluminium, de cobalt et de molybdène (OBOLENTSEV R.D. Khim. Seroorgan. Soedin. Soderzhasch. v. Nelft. 1 Nefteprod. Akad. Nauk SSSR, Bashkirsk. Filial 4-245-255-1961 et Bashkirsk. Filial 7-148-155-1964), mais les bons rendements sont obtenus dans les premiers instants de la réaction et ne sont pas constants. L'activité du catalyseur baisse très vite à cause du cokage qui bloque la réaction de déshydrogénation.

La déshydrogénation oxydante du THT par le $SO_2$ en présence d'un catalyseur (oxyde métallique, alumine activée ou charbon actif) est décrite dans le brevet FR 2 071 189. Il y est indiqué que les meilleurs résultats sont obtenus surtout entre 500 et 600 °C et que, lorsque la température est trop basse, la désactivation du catalyseur est importante, principalement par suite de condensation de soufre, et que de plus le décokage est incomplet.

Dans l'article intitulé "Study of the mechanism of thiophene formation in the presence of sulfide catalysts" (Geterog. Katal. 1979, 4th, Pt. 2, 237-42 ; Chemical Abstracts, vol. 93, 131779 d), T.S. SUKHAREVA et al. indiquent que les catalyseurs les plus actifs sont les sulfures de rhénium, molybdène, fer et platine.

Il a maintenant été trouvé que le sulfure de ruthénium est beaucoup plus actif et très sélectif dans la déshydrogénation du THT. L'utilisation d'un catalyseur à base de sulfure de ruthénium permet d'effectuer la déshydrogénation à des températures relativement basses et de stabiliser l'activité du catalyseur sur une plus longue période.

L'invention a donc pour objet un procédé de fabrication du thiophène par déshydrogénation catalytique du THT, caractérisé en ce qu'on fait passer un courant gazeux de THT sur un catalyseur à base de sulfure de ruthénium.

Ce sulfure est très actif à l'état massique. Cependant, pour des raisons de commodité d'emploi à l'échelle industrielle, on préfère l'utiliser sous forme de catalyseur supporté dont la teneur en ruthénium peut aller de 2 à 60 % en poids, de préférence de 2 à 20 % et plus particulièrement de 5 à 15 %. Le support peut être, par exemple, une alumine, une silice, le kieselguhr, l'oxyde de titane ou de zirconium, une silice-alumine, l'oxyde de thorium ou un charbon actif.

On ne sortirait pas du cadre de la présente invention en utilisant un mélange de sulfures de ruthénium et d'au moins un autre métal de transition choisi dans le groupe constitué par les métaux suivants : vanadium, chrome, molybdène, rhodium, palladium, tungstène, manganèse, niobium, nickel, fer ou cobalt, la proportion de métal autre que Ru dans le mélange pouvant aller jusqu'à 80 % at.

La préparation des catalyseurs, supportés ou non, peut être effectuée suivant différentes méthodes connues en soi telles que, par exemple, les suivantes :

- Catalyseurs massiques : précipitation par addition de sulfure de sodium à une solution méthanolique de chlorure(s) métallique(s), puis extraction du chlorure de sodium et enfin traitement du solide à une température comprise entre 300 et 600 °C, de préférence à environ 400 °C en présence d'un mélange d'hydrogène et de $H_2S$ (2 à 50 % en volume, de préférence environ 15 %).
- Catalyseurs supportés : imprégnation du support avec un ou des sel(s) métallique(s), par exemple chlorure(s), puis sulfuration directe par un mélange d'hydrogène et de $H_2S$ (2 à 50 % en volume, de préférence environ 15 %) ou par $H_2S$ dilué dans un gaz inerte (par exemple l'azote) à une température comprise entre 300 et 700 °C, préférentiellement entre 400 et 600 °C et plus particulièrement à environ 500 °C.

Les traitements en température par les mélanges $H_2S$ + diluant (hydrogène ou gaz inerte) sont de préférence effectués dans le réacteur de production de thiophène, après y avoir placé le catalyseur mis sous une forme convenable, par exemple, sous forme de pastilles obtenues selon des techniques connues en soi.

Les conditions dans lesquelles est effectuée la réaction de déshydrogénation du THT peuvent varier dans de larges limites sans nuire à l'obtention d'un rendement élevé en thiophène, dès lors que l'on se place à une température et à des pressions partielles en THT et $H_2S$ quine limitent pas le déplacement de l'équilibre thermodynamique vers la formation totale de thiophène. On peut opérer à une température comprise entre 300 et 600 °C, mais on opère de préférence entre 350 et 450 °C et plus particulièrement entre environ 380 et 420 °C. La réaction peut être effectuée à pression atmosphérique ou à une pression supérieure allant jusqu'à 15 bars absolus. Le temps de contact peut varier entre 0,1 et 10 secondes et est de préférence compris entre 1 et 3 secondes.

De façon à limiter la pression partielle du THT dans le flux gazeux passant sur le catalyseur, on peut utiliser des diluants inertes tels que, par exemple, les gaz rares ou l'azote. Cependant, on préfère diluer le THT avec $H_2S$ dans un rapport molaire $H_2S$/THT allant de 0,1 à 10, de préférence 1 à 5, et surtout d'environ 4.

Les exemples suivants illustrent l'invention, sans la limiter.

## EXEMPLE 1

Une série de sulfures des métaux de transition non supportés a été préparée en utilisant les modes opératoires décrits dans la littérature (J. Catal 120, 473, 1989).

Les sulfures ainsi synthétisés ont été testés dans la réaction de déshydrogénation du tétrahydrothiophène dans les conditions suivantes :

| | |
|---|---|
| Température | 380 °C |
| Pression totale | 1 bar |
| Pression partielle de THT | 6,6 $10^2$Pa |
| Pression partielle de $H_2S$ | 6,6 $10^2$Pa |

On a utilisé un microréacteur tubulaire en verre de 30 ml de volume, contenant une masse de catalyseur comprise entre 50 et 300 mg et travaillant en continu, le débit global étant généralement de 60 ml. Le THT est introduit dans l'enceinte réactionnelle par l'intermédiaire d'un saturateur-condenseur maintenu à température constante, cette dernière fixant la pression partielle de THT. L'analyse des effluents gazeux, issus du réacteur, est effectuée par chromatographie en phase gazeuse.

Les activités catalytiques déterminées à l'état pseudo-stationnaire sont résumées dans le tableau suivant. L'activité du sulfure de ruthénium selon l'invention s'avère très supérieure à celles des autres sulfures métalliques testés.

## TABLEAU 1

Propriétés catalytiques des sulfures des métaux de transition non supportés dans la réaction de déshydrogénation de tétrahydrothiophène.

| Sulfure du métal | Activité spécifique $10^{-8}$mol $s^{-1}g^{-1}$ | Surface spécifique m²/g | Activité intrinsèque $10^{-8}$mol $s^{-1}m^{-2}$ | Sélectivité en thiophène % |
|---|---|---|---|---|
| V | 27,6 | 46 | 0,6 | 93 |
| Cr | 172 | 160 | 1,1 | 94 |
| Mn | 1,4 | 6 | 0,2 | 62 |
| Fe | 3 | 5 | 0,6 | 60 |
| Co | 8 | 28 | 0,3 | 80 |
| Nb | 4 | 15 | 0,3 | 86 |
| Mo | 12 | 8 | 1,5 | 96 |
| Ru | 490 | 40 | 12,3 | 97 |
| Rh | 56 | 9,3 | 6 | 96 |
| Pd | 24 | 14 | 1,7 | 90 |
| W | 25 | 17,6 | 1,4 | 92 |
| Zn | 5,2 | 11 | 0,5 | 24 |
| Ni | ε | - | ε | - |
| Cd | ε | - | ε | - |

## EXEMPLE 2

On a préparé trois catalyseurs supportés sur alumine en imprégnant une alumine de transition avec différents sels précurseurs de sulfure de vanadium, de molybdène ou de ruthénium. Pour les catalyseurs à base de vanadium et de molybdène, on a utilisé respectivement le thiovanadate d'ammonium et l'heptamolybdate d'ammonium. Pour le catalyseur à base de sulfure de ruthénium selon l'invention, on a utilisé le chlorure de ruthénium qui permet d'obtenir une meilleure dispersion et une plus grande sulfurabilité du ruthénium.

L'alumine utilisée est une alumine gamma-cubique, préformée sous forme d'extrudés de 1,2 mm. Sa surface spécifique est de 232 $m^2$ /g et ce support présente un volume poreux de 0,64 $cm^3$/g. La perte au feu à 1000 °C est de 2,1 %.

Ce support, préalablement séché à une température comprise entre 100 et 250 °C, de préférence à 180 °C, est mis en contact avec une solution aqueuse du sel métallique correspondant au catalyseur désiré. La quantité d'eau utilisée est égale au volume poreux du support, mais un excès d'eau pouvant aller

jusqu'au triple de celui nécessaire au remplissage des pores du support peut être éventuellement utilisé sans inconvénient.

Après séchage entre 100 et 200 °C, les précurseurs ont été sulfurés par traitement à 400 °C pendant 4 heures au moyen d'un mélange de $H_2S$ et d'azote (cas de Ru) ou d'hydrogène (cas de Mo et V), la teneur en $H_2S$ étant de l'ordre de 15 % en volume.

Les catalyseurs supportés ainsi obtenus ont ensuite été testés dans la réaction de déshydrogénation du THT dans les mêmes conditions qu'à l'exemple 1. Les résultats obtenus sont rassemblés dans le tableau 2 suivant dont la seconde colonne indique la teneur pondérale en métal (Mo, V ou Ru) du catalyseur supporté.

**TABLEAU 2**

**Propriétés des sulfures supportés sur alumine.**

| Catalyseur | % métal | Activité spécifique $10^{-7}$ mol $s^{-}g^{-1}$ | Sélectivité en thiophène % |
|---|---|---|---|
| $Mo/Al_2O_3$ | 10,6 | 1,2 | 70 |
| $V/Al_2O_3$ | 9 | 2,4 | 80 |
| $Ru/Al_2O_3$ | 7 | 18 | 92 |

Ces résultats montrent que les excellentes propriétés déshydrogénantes obtenues sur le sulfure de ruthénium non supporté sont maintenues lorsque le sulfure de ruthénium est dispersé sur un support tel que l'alumine.

## EXEMPLE 3

L'influence des conditions d'activation du catalyseur $Ru/Al_2O_3$ (7 % Ru en poids) a été étudiée en faisant le test catalytique dans les mêmes conditions opératoires que celles utilisées dans l'exemple précédent. Les activités et sélectivités obtenues pour différentes températures d'activation du catalyseur par un mélange $N_2/H_2S$ (15 % $H_2S$ Vol) sont reportées dans le tableau 3 suivant :

**TABLEAU 3**

**Activités et sélectivités en fonction de la température d'activation.**

| Température d'activation | Activité spécifique $10^{-7}$mol/s/g | Sélectivité en thiophène % |
|---|---|---|
| 400°C | 18 | 92 |
| 500°C | 40 | 90 |
| 600°C | 30 | 85 |

## EXEMPLE 4

Les résultats reportés dans les exemples 1 et 2 montrent la supériorité de la phase pyrite $RuS_2$ supportée ou non. Le catalyseur $RuS_2/Al_2O_3$ contenant 7 % de Ru en poids a donc été testé en micropilote dans les conditions opératoires suivantes:

| | |
|---|---|
| Flux de THT | 0,33 mol/h |
| Flux d'$H_2S$ | variable |
| Masse de catalyseur | 38 g |
| Température de réaction | 380 °C |
| Pression totale | 1 bar |

Le rôle de l'ajout $H_2S$ est, d'une part, de limiter les réactions secondaires conduisant à la formation d'hydrocarbures par rupture de la liaison C-S et, d'autre part, de diluer le réactif afin d'accroître la conversion limite imposée par la thermodynamique. De plus, la présence d'hydrogène sulfuré permet de maintenir le catalyseur à l'état de sulfure.

Le réacteur utilisé est un réacteur à lit fixe constitué par un tube vertical en acier inox 316 de 800 mm de longueur et de 25 mm de diamètre, muni d'une gaine thermométrique permettant un contrôle précis du profil de température le long du lit catalytique. Le THT est introduit à l'état liquide par l'intermédiaire d'une pompe et rencontre l'$H_2S$ dans un préchauffeur, situé en amont du réacteur et maintenu à 150 °C pour permettre la volatilisation rapide du THT. En sortie du réacteur, la phase liquide est récupérée dans des condenseurs.

L'influence du rapport molaire $H_2S$/THT sur la sélectivité en thiophène a été étudiée dans des conditions opératoires conduisant à une conversion globale de l'ordre de 30 %. Le tableau 4 résume les résultats obtenus.

### TABLEAU 4

**Effet du rapport $H_2S$/THT sur la distribution des produits de la réaction.**

| Rapport molaire $H_2S$/THT | Conversion globale % | Sélectivité | | |
|---|---|---|---|---|
| | | C1+C2+C3 % | C4 % | Thiophène % |
| 1 | 35 | 10 | 30 | 60 |
| 1,6 | 32 | 7 | 26 | 67 |
| 2 | 31 | 5 | 21 | 74 |
| 4 | 32 | 4 | 18 | 78 |

## EXEMPLE 5

Le comportement du catalyseur $RuS_2/Al_2O_3$ au cours du temps a été étudié dans les mêmes conditions opératoires qu'à l'exemple précédent en utilisant un rapport $H_2S$/THT égal à 2. L'évolution de la conversion et de la sélectivité en thiophène en fonction du temps de travail du catalyseur est donnée dans le tableau suivant :

**TABLEAU 5**

Etude du vieillissement du catalyseur $RuS_2/Al_2O_3$.

| Temps (h) | Conversion globale (%) | Sélectivité en thiophène (%) |
|---|---|---|
| 25 | 90 | 74 |
| 35 | 86 | 74 |
| 80 | 77 | 75 |
| 100 | 74 | 74 |
| 120 | 71 | 73 |
| 140 | 61 | 74 |
| 165 | 55 | 73 |
| 200 | 49 | 74 |
| 250 | 34 | 73 |
| 320 | 16 | 74 |

Ces résultats indiquent que le catalyseur se désactive linéairement au cours du temps de travail sans qu'il soit observé de modification de sélectivité. Dans les conditions opératoires utilisées, le temps de 1/2 vie du solide est de l'ordre de 200 heures.

**EXEMPLE 6**

Le catalyseur issu de l'exemple précédent contient une proportion de "coke" de l'ordre de 17 % en poids. Pour éliminer ce dernier, on soumet le catalyseur usé à un traitement de régénération avec de l'air ou avec un mélange d'air et d'eau (rapport molaire air/eau = 20). Ce traitement oxydant est effectué à 350 °C avec un temps de contact catalyseur-air (ou air + eau) d'environ 3 secondes.

A l'issue de ce traitement, les phases sulfures, transformées en oxydes ou en oxosulfures, sont resulfurées par traitement au moyen d'un mélange $H_2S/N_2$ dans les mêmes conditions qu'à l'exemple 2.

Les résultats obtenus avec les catalyseurs régénérés sont reportés dans le tableau suivant :

**TABLEAU 6**

**Propriétés catalytiques des catalyseurs régénérés.**

| Atmosphère de régénération | Conversion initiale (25 heures) | Conversion après 150 heures | Sélectivité en thiophène % |
|---|---|---|---|
| air | 85 % | 55 % | 74 |
| air + eau | 88 % | 60 % | 74 |

D'une façon générale, le traitement de régénération peut être réalisé à une température comprise entre 300 et 600 °C, de préférence entre 350 et 400 °C, avec un temps de contact compris entre 0,1 et 10 secondes. Lorsqu'on utilise un mélange air + eau, le rapport molaire air/eau peut aller de 1 à 40.

**Revendications**

**1.** Procédé de fabrication du thiophène par déshydrogénation catalytique du tétrahydrothiophène, caractérisé en ce qu'on fait passer un courant de tétrahydrothiophène gazeux sur un catalyseur à base de sulfure de ruthénium.

**2.** Procédé selon la revendication 1, dans lequel le sulfure de ruthénium est employé à l'état massique.

**3.** Procédé selon la revendication 1, dans lequel le sulfure de ruthénium est déposé sur un support.

**4.** Procédé selon la revendication 3, dans lequel le support est une alumine, une silice, le kieselguhr, l'oxyde de titane ou de zirconium, une silice-alumine, l'oxyde de thorium ou un charbon actif.

**5.** Procédé selon la revendication 3 ou 4, dans lequel la teneur en ruthénium du catalyseur supporté est comprise entre 2 et 60 % en poids, de préférence entre 2 et 20 %, et plus particulièrement entre 5 et 15 %.

**6.** Procédé selon l'une des revendications 1 à 5, dans lequel la déshydrogénation est effectuée à une température comprise entre 300 et 600 °C, de préférence entre 350 et 450 °C, et plus particulièrement entre environ 380 et 420 °C.

**7.** Procédé selon l'une des revendications 1 à 6, dans lequel on effectue la réaction à pression atmosphérique ou à une pression pouvant aller jusqu'à 15 bars absolus.

**8.** Procédé selon l'une des revendications 1 à 7, dans lequel le tétrahydrothiophène est dilué avec un gaz inerte ou, de préférence, avec de l'hydrogène sulfuré dans un rapport molaire $H_2S$/tétrahydrothiophène allant de 0,1 à 10, de préférence 1 à 5, et surtout d'environ 4.

**9.** Procédé selon l'une des revendications 1 à 8, dans lequel on utilise un mélange de sulfures de ruthénium et d'au moins un métal de transition choisi dans le groupe constitué par les métaux suivants : vanadium, chrome, molybdène, rhodium, palladium, tungstène, manganèse, niobium, nickel, fer et cobalt, la proportion de métal autre que le ruthénium dans le mélange pouvant aller jusqu'à 80 % at.

## Claims

1. Process for the manufacture of thiophene by catalytic dehydrogenation of tetrahydrothiophene, characterized in that a stream of gaseous tetrahydrothiophene is passed over a catalyst based on ruthenium sulphide.

2. Process according to Claim 1, in which the ruthenium sulphide is employed in bulk form.

3. Process according to Claim 1, in which the ruthenium sulphide is deposited on a support.

4. Process according to Claim 3, in which the support is an alumina, a silica, kieselguhr, titanium or zirconium oxide, a silica-alumina, thorium oxide or an active carbon.

5. Process according to Claim 3 or 4, in which the ruthenium content of the supported catalyst is between 2 and 60 % by weight, preferably between 2 and 20 %, and more particularly between 5 and 15 %.

6. Process according to one of Claims 1 to 5, in which the dehydrogenation is performed at a temperature of between 300 and 600 °C, preferably between 350 and 450 °C, and more particularly between approximately 380 and 420 °C.

7. Process according to one of Claims 1 to 6, in which the reaction is performed at atmospheric pressure or at a pressure which can range up to 15 bars absolute.

8. Process according to one of Claims 1 to 7, in which the tetrahydrothiophene is diluted with an inert gas or, preferably, with hydrogen sulphide in an $H_2S$/tetrahydrothiophene molar ratio ranging from 0.1 to 10, preferably 1 to 5, and especially of approximately 4.

9. Process according to one of Claims 1 to 8, in which use is made of a mixture of sulphides of ruthenium and of at least one transition metal chosen from the group consisting of the following metals: vanadium, chromium, molybdenum, rhodium, palladium, tungsten, manganese, niobium, nickel, iron and cobalt, it being possible for the proportion of the metal other than ruthenium in the mixture to range up to 80 at%.

## Patentansprüche

1. Verfahren zur Herstellung von Thiophen durch katalytische Dehydrierung von Tetrahydrothiophen, dadurch gekennzeichnet, daß man einen Strom aus gasförmigem Tetrahydrothiophen über einen Katalysator auf Rutheniumsulfidbasis leitet.

2. Verfahren nach Anspruch 1, dadurch gekennzeichnet, daß das Rutheniumsulfid in reiner Form verwendet wird.

3. Verfahren nach Anspruch 1, dadurch gekennzeichnet, daß das Rutheniumsulfid auf einem Träger abgeschieden wird.

4. Verfahren nach Anspruch 3, dadurch gekennzeichnet, daß der Träger ein Aluminiumoxid, ein Siliziumoxid, Kieselgur, Titan- oder Zirkoniumoxid, ein Siliziumoxid-Aluminiumoxid, ein Thoriumoxid oder Aktivkohle ist.

5. Verfahren nach Anspruch 3 oder 4, dadurch gekennzeichnet, daß der Rutheniumgehalt des Trägerkatalysators im Bereich von 2 bis 60 Gew.%, vorzugsweise 2 bis 20 Gew.% und insbesondere von 5 bis 15 Gew.% liegt.

6. Verfahren nach einem der Ansprüche 1 bis 5, dadurch gekennzeichnet, daß die Dehydrierung bei einer Temperatur im Bereich von 300 bis 600 °C, vorzugsweise von 350 bis 450 °C und insbesondere von etwa 380 bis 420 °C durchgeführt wird.

**7.** Verfahren nach einem der Ansprüche 1 bis 6, dadurch gekennzeichnet, daß die Reaktion bei Atmosphärendruck oder einem Druck bis zu 15 bar abs. durchgeführt wird.

**8.** Verfahren nach einem der Ansprüche 1 bis 7, dadurch gekennzeichnet, daß das Tetrahydrothiophen mit einem inerten Gas oder vorzugsweise mit Schwefelwasserstoff in einem molaren Verhältnis von $H_2S$/Tetrahydrothiophen von 0,1 bis 10, vorzugsweise 1 bis 5, und insbesondere etwa 4 verdünnt wird.

**9.** Verfahren nach einem der Ansprüche 1 bis 8, dadurch gekennzeichnet daß ein Gemisch aus Rutheniumsulfiden und wenigstens einem Übergangsmetall verwendet wird, welches ausgewählt wird aus der Gruppe bestehend aus den folgenden Metallen. Vanadium, Chrom, Molybdän, Rhodium, Palladium, Wolfram, Mangan, Niob, Nickel, Eisen und Kobalt, wobei der Gehalt des Metalls außer Ruthenium in der Mischung bis zu 80 % betragen kann.